# EUROPEAN PATENT SPECIFICATION

(11) **EP 0 539 651 B1**
(45) Date of publication and mention of the grant of the patent: **03.04.1996**
(21) Application number: 92106549.6
(22) Date of filing: 15.04.1992
(51) Int. Cl.: A61K 7/16

(54) **Dentifrice containing antibacterial material**
Zahnpflegemittel, das antibakterielle Mittel enthält
Dentifrice contenant un agent antibactériel

(30) Priority: 29.10.1991 JP 282636/91
(43) Date of publication of application: 05.05.1993
(73) Proprietor: SANGI CO., LTD., Chuo-ku, Tokyo (JP)
(72) Inventor: Sakuma, Shuji, c/o Sangi Co., Ltd., Chuo-ku, Tokyo (JP); Atsumi, Kiminori, c/o Sangi Co., Ltd., Chuo-ku, Tokyo (JP)
(74) Representative: Grünecker, Kinkeldey, Stockmair & Schwanhäusser Anwaltssozietät

(56) References cited:
- EP-A- 0 428 493
- US-A- 3 989 814

## Description

This invention relates to improvements in a dentifrice (tooth paste or powder), and more particularly to the dentifrice having an antibacterial effect.

In general, a dentifrice (tooth paste or powder) includes abrasive powder, foaming agent, perfume and taste adjusting agents, lubricant, caking additive and medicative ingredients as main components. In recent years, a dentifrice provided with an antibacterial effect has been developed, in which organic substances such as parahydroxybenzoates, chlorhexidines, hinokitiol and protamine and inorganic substances such as salt are known and used as materials exhibiting an antibacterial effect.

However, such a dentifrice containing the antibacterial materials is problematic from view points of toxicity, stability and taste, owing to the character of the antibacterial materials. Particularly, almost all of the conventional antibacterial materials are low in heat-resistance and soluble in water, and therefore difficult to be thermally processed and not durable to use for a long period of time.

In this connection, it is known that metals such as silver, copper and zinc and salts thereof exhibit a high antibacterial effect. However, these metals or the salts cannot be safely used in a dentifrice (tooth paste) because ions of these metals tend to be readily released thereby causing a toxicity.

US-A-3989814 provides a dentifrice possessing enhanced polishing characteristics containing an abrasive system including calcium pyrophosphate and a non-toxic zink compound Said system reduces enamel abrasivity.

It is the object of the present invention to provide an improved dentifrice (tooth paste or powder) which can overcome the drawbacks encountered in conventional dentifrices, which is high in safety and stability, while maintaining a high antibacterial effect, and wherein an antibacterial material is stably and firmly carried or supported by a carrier as a component of the dentifrice.

According to the present invention this object is achieved by a dentifrice (tooth paste or powder) comprising:
at least one calcium compound selected from the group consisting of calcium hydrogenphosphate, tricalcium phosphate, calcium diphosphate, calcium carbonate, and apatite, said calcium compound being in the form of a powder; and
at least one antibacterial metal selected from the group consisting of silver, copper and zinc, which is carried by said calcium compound powder by ion exchange and/or adsorption. Accordingly, the dentifrice exhibits a high antibacterial effect. The antibacterial metal carried by the calcium compound is highly stable, and therefore the toxicity due to the ion form of the metal is prevented while contributing to a medical treatment of teeth and further providing the dentifrice with a good preservability for a long period of time. Thus, the dentifrice of the present invention is highly effective for preventing occurrence of carious tooth and periodontal diseases such as alveolar blennorrhea.

The calcium compounds used in the present invention are selected from calcium hydrogenphosphate [CaHPO₄·0-2H₂0], tricalcium phosphate [Ca₃(PO₄)₂], calcium diphosphate [Ca₂P₂0₇], calcium carbonate [CaCO₃] and apatite, such as hydroxylapatite. The hydroxylapatite is preferably in the form of a powder. The antibacterial metal(s) are carried by or combined to the calcium compound under conditions of ion exchange and/or adsorption, thereby forming antibacterial calcium compounds(s). The antibacterial calcium compound(s) is mixed in the dentifrice (tooth paste or powder). The use of the antibacterial metal adds the functions of preventing generation of carious tooth and periodontal diseases such as alveolar blennorrhea, to the dentifrice.

A method of production of an antibacterial calcium hydrogenphosphate will be discussed. First, metallic salt(s) such as silver nitrate or silver sulfate is added to an aqueous suspension of calcium hydrogenphosphate under agitation to promote the reaction between the metallic salt(s)and the calcium hydrogenphosphate, thereby forming a reaction product. Thereafter, the reaction product is rinsed, dehydrated and dried thereby to obtain the antibacterial calcium hydrogenphosphate. It will be understood that antibacterial tricalcium phosphate, antibacterial calcium diphosphate, antibacterial calcium carbonate and antibacterial apatite can be obtained by the same production method as that of the antibacterial calcium hydrogenphosphate.

The amount of the antibacterial metal carried by the calcium compound is suitably selected according to the kind of the metallic salt to be used, the concentration and the temperature of the aqueous suspension of the calcium compound. The amount of the antibacterial metallic salt is preferably not more than 30% by weight, and more preferably 5 to 0.0001% by weight relative to the amount of the calcium compound such as calcium phosphate and calcium carbonate.

The thus obtained antibacterial calcium compound exhibits a sufficient antibacterial effect even upon addition of a small amount thereof to the dentifrice, maintaining the antibacterial effect for a long period of time. Additionally, the antibacterial calcium compound can be safely used since the amount of released metal to water is extremely small, i.e., less than several ppb.

The dentifrice of the present invention will be discussed further in detail with reference to experiments for Examples and Comparative Examples.

### Experiment 1

### [Preparation of Specimen Tooth Paste and Powder]

In this experiment, first specimen tooth pastes having compositions of Examples 1 to 4 and of Comparative Example 1 were prepared.

### EXAMPLE 1 (tooth paste)

| | |
|---|---|
| Antibacterial calcium hydrogenphosphate (silver 0.1 wt% contained) | 41.0 wt% |
| Glycerine | 20.0 wt% |
| Carageenan | 1.1 wt% |
| Sodium lauryl sulfate | 1.5 wt% |
| Saccharin | 1.0 wt% |
| Perfume | 1.0 wt% |
| Butyl para-hydroxybenzoate | 0.005 wt% |
| Water | balance |

### EXAMPLE 2 (tooth paste)

| | |
|---|---|
| Antibacterial tricalcium phosphate (silver 2 wt% and zinc 3 wt% contained) | 3.0 wt% |
| Aluminum hydroxide | 37.0 wt% |
| Carboxymethyl cellulose | 1.3 wt% |
| Sorbitol | 19.0 wt% |
| Propylene glycol | 2.0 wt% |
| Sodium lauryl sulfate | 1.0 wt% |
| Sodium saccharin | 1.0 wt% |
| Perfume | 1.1 wt% |
| Butyl para-hydroxybenzoate | 0.005 wt% |
| Water | balance |

### EXAMPLE 3 (tooth paste)

| | |
|---|---|
| Antibacterial calcium diphosphate (silver 1 wt% and Zinc 2 wt% contained) | 42.0 wt% |
| Calcium laury sulfate | 1.2 wt% |
| Sodium lauroyl sarcosinate | 0.2 wt% |
| Glycerine | 20.0 wt% |
| Carageenan | 1.1 wt% |
| Ester of saccharose fatty acid | 2.0 wt% |
| Perfume | 1.2 wt% |
| Water | balance |

### EXAMPLE 4 (tooth powder)

| | |
|---|---|
| Antibacterial calcium carbonate (silver 0.001 wt% contained) | 75.0 wt% |
| Grycerine | 10.0 wt% |
| Perfume | 1.0 wt% |
| Butyl para-hydroxylbenzoate | 0.005 wt% |
| Sodium lauryl sulfate | 1.3 wt% |
| Saccharin | 0.1 wt% |
| Water | balance |

### COMPARATIVE EXAMPLE 1 (tooth paste)

| | |
|---|---|
| Calcium hydrogenphosphate | 41.0 wt% |
| Glycerine | 20.0 wt% |
| Carageenan | 1.1 wt% |
| Sodium lauryl sulfate | 1.5 wt% |
| Saccharin | 1.0 wt% |
| Perfume | 1.0 wt% |
| Butyl para-hydroxybenzoate | 0.005 wt% |
| Water | balance |

### [Antibacterial Test]

1 g of each of each specimen tooth paste prepared in Examples 1 to 4 and Comparative Example 1 was added to 10 ml of a liquid containing one kind of the following bacilli in a flask: streptococcus mutans type G and streptococcus sanguis as bacilluses or causatives for carious tooth, and actinobacillus actinomycetemcomitans Y4 and bacteroides gingivalis as bacillus or causatives for periodontal diseases. The flask was shaked according to a so-called shake flask method in which the number of living bacilli was measured with lapse of time in order to evaluate an antibacterial effect. The result of the antibacterial test is shown in Table 1.

**TABLE 1**

| Bacillus | Sample | Living bacilli (number/ml) | | |
|---|---|---|---|---|
| | | 0 h lapsed | 6 h after | 12 h after |
| Streptococcus mutans type G | Example 1 | 6.8×10⁵ | <1 | <1 |
| | Example 2 | 6.8×10⁵ | <1 | <1 |
| | Example 3 | 6.8×10⁵ | <1 | <1 |
| | Example 4 | 6.8×10⁵ | <1 | <1 |
| | Comparative Example 1 | 6.8×10⁵ | 9.5×10⁴ | 4.1×10⁵ |
| Streptococcus sanguis | Example 1 | 4.0×10⁵ | 8.2×10² | <1 |
| | Example 2 | 4.0×10⁵ | <1 | <1 |
| | Example 3 | 4.0×10⁵ | 3.3×10 | <1 |
| | Example 4 | 4.0×10⁵ | 3.7×10² | 4.9×10 |
| | Comparative Example 1 | 4.0×10⁵ | 5.7×10⁵ | 6.4×10⁵ |
| Actinobacillus actinomycetemcomitans Y4 | Example 1 | 3.7×10⁵ | 2.4×10 | <1 |
| | Example 2 | 3.7×10⁵ | <1 | <1 |
| | Example 3 | 3.7×10⁵ | <1 | <1 |
| | Example 4 | 3.7×10⁵ | 1.5×10² | <1 |
| | Comparative Example 1 | 3.7×10⁵ | 2.9×10⁵ | 2.2×10⁵ |
| Bacteroides gingivalis | Example 1 | 6.5×10⁵ | 1.8×10² | <1 |
| | Example 2 | 6.5×10⁵ | 1.7×10 | <1 |
| | Example 3 | 6.5×10⁵ | <1 | <1 |
| | Example 4 | 6.5×10⁵ | 5.1×10³ | 2.6×10 |
| | Comparative Example 1 | 6.5×10⁵ | 8.4×10⁵ | 1.5×10⁵ |

As can be appreciated from Table 1, the specimen tooth pastes of Examples 1 to 4 (according to the present invention) are considerably high in antibacterial effect to the bacilli for carious tooth and periodontal diseases such as alveolar blennorrhea, as compared with that of Comparative Example 1 (a conventional tooth paste). Additionally, a released metal content test for the specimen tooth pastes was conducted, which revealed that no releasing of the antibacterial metal was recognized.

While only silver and zinc have been described as being used as the antibacterial metal in Examples, it will be appreciated that they may be replaced with other antibacterial metals.

The above-mentioned examples of the calcium compound include apatite. In case of using apatite as the calcium compound of the present invention, the antibacterial metal such as silver (Ag), copper (Cu) and/or zinc (Zn) is carried by or combined with the apatite under ion exchange and/or adsorption thereby to form an antibacterial apatite. The thus formed antibacterial apatite e.g takes the form of Ca₁₀₋ₓ ·Mₓ(PO₄)₅(OH)₂, Ca₁₀(M·PO₄)₅(OH)₂, in which M is the antibacterial metal, and x is an integer or decimal larger than 0. The antibacterial apatite is mixed in the dentifrice (tooth paste). The antibacterial apatite is stable and heat-resistive, and good in mixability with the tooth paste since the apatite is in the state of powder.

The antibacterial apatite can be easily produced under coexistence of the metallic salt of the antibacterial metal(s) during synthesis of the apatite, or by reacting the metallic salt with the apatite. More specifically, according to the former method, a phosphoric acid aqueous solution is dropped to an aqueous solution containing calcium hydroxide and the metallic salt(s) of silver, copper and/or zinc. under stirring thereby synthesizing the antibacterial apatite. According to the latter method, fine powder of the apatite obtained under the conventional method is suspended in distilled water to form a suspension. To the suspension, a water-soluble metallic salt(s) of the antibacterial metal(s) is added and then stirred to promote the reaction between the apatite and the antibacterial metal(s) thereby to form a product. Thereafter, the product is rinsed, dried and pulverized thus obtaining the antibacterial apatite.

In the above production processes, there is the possibility that calcium salt remains in the resultant antibacterial apatite, the calcium salt being formed during the reaction process in which acidic roots, the metallic salts and metallic ions are replaced with calcium ions. Accordingly, it is preferable that the resultant antibacterial apatite is sufficiently rinsed with water in order to completely remove these foreign matters.

The amount of the antibacterial metal carried by the apatite is suitably selected according to the kind of the antibacterial metal to be used, and the concentration and the temperature of the aqueous solution containing the apatite or raw materials of the apatite. The amount of the antibacterial metal is preferably not more than 30% by weight, more preferably 5 to 0.0001% by weight relative to the apatite.

The thus obtained antibacterial apatite exhibits a sufficient antibacterial effect even upon addition of a small amount thereof to the dentifrice, maintaining the antibacterial effect for a long period of time. Additionally, the antibacterial apatite can be safely used since the amount of released metal to water is very small, i.e., less than several ppb.

### EXPERIMENT 2

The antibacterial apatite will be discussed further in detail with reference to experiments conducted to evaluate advantageous effects of' the antibacterial apatite.

### [Preparation of Specimen Tooth Paste]

Specimen tooth pastes having compositions of Examples 5 and 6, Reference Example 1 and Comparative Example 2 were prepared. In Example 5, the antibacterial apatite was a hydroxylapatite carrying 2% by weight of silver and 3% by weight of zinc which were adsorbed to or combined (under ion exchange) with the apatite. The antibacterial hydroxylapatite obtained by ion exchange e.g. seems to take the form of Ca_{10-x-y}· Zn_{y}·Agₓ(PO₄)₆(OH)₂ or Ca_{10-y}·Zn_{y}(Ag·PO₄)₆(OH)₂, in which x and y are integers or decimals, respectively, larger than 0. In Example 6, the antibacterial apatite was a hydroxylapatite carrying 0.1% wt weight of silver which had been absorbed to and carried (under ion exchange) by the hydroxylapatite. The antibacterial hydroxylapatite obtained by ion exchange e.g. seems to take the form of Ca₁₀₋ₓ· Agₓ(PO₄)₆(0H)₂, Ca₁₀(Ag·PO₄)₆(OH)₂.

### EXAMPLE 5

| | |
|---|---|
| Antibacterial hydroxylapatite | 5.0 wt% |
| Calcium phosphate | 10.0 wt% |
| Calcium pyrophosphate | 20.0 wt% |
| Sodium salt CMC | 1.0 wt% |
| Sodium alginate | 0.1 wt% |
| Sorbitol | 10.0 wt% |
| Sodium lauryl sulfate | 1.5 wt% |
| Lauroyl sarcosine sodium | 0.5 wt% |
| Perfume | 0.5 wt% |
| Sodium saccharin | 0.1 wt% |
| Silicon nitride | 2.5 wt% |
| Citric acid | 2.0 wt% |
| Sodium phosphate | 1.0 wt% |
| Water | 35.0 wt% |

### EXAMPLE 6

| | |
|---|---|
| Antibacterial hydroxylapatite | 50.0 wt% |
| Calcium phosphate | 25.0 wt% |
| Sodium salt CMC | 0.3 wt% |
| Carageenan | 1.2 wt% |
| Glycerine | 5.0 wt% |

### REFERENCE EXAMPLE 1

Antibacterial hydroxylapatite (powder) carrying 1% by weight of silver (Ag) and 0.3% by weight of copper (Cu) 100 wt%

### COMPARATIVE EXAMPLE 2

| | |
|---|---|
| Hydroxylapatite | 5.0 wt% |
| Calcium phosphate | 10.0 wt% |
| Calcium pyrophosphate | 20.0 wt% |
| Sodium salt CMC | 1.0 wt% |
| Sodium alginate | 0.1 wt% |
| Sorbitol | 10.0 wt% |
| Sodium lauryl sulfate | 1.5 wt% |
| Lauroyl sarcosine sodium | 0.5 wt% |
| Perfume | 0.5 wt% |
| Sodium saccharin | 0.1 wt% |
| Silicon dioxide | 2.5 wt% |
| Citric acid | 2.0 wt% |
| Sodium phosphate | 1.0 wt% |
| Water | 35.0 wt% |

### [Antibacterial Test (I)]

In case of Examples 5 and 6 and Comparative Example 2, 1 g of each specimen tooth paste was sampled. In case of Reference Example 1, 0.001 g of the powder of the antibacterial hydroxylapatite was sampled. Each of the thus sampled specimen tooth paste and the hydroxylapatite powder was added to 10 ml of a liquid containing one kind of the following bacilli in a flask:
streptococcus mutans type G and streptococcus sanguis as bacillus or causatives for carious tooth, and actinobacillus actinomycetemcomitans Y4 and bacteroides gingivalis as bacillus or causatives for periodontal diseases.

The flask was shaked according to the shake flask method in which the number of living bacilli was measured with lapse of time in order to evaluate an antibacterial effect. The result of the antibacterial test is shown in Table 2.

**TABLE 2**

| Bacillus | Sample | Living bacilli (number/ml) | | |
|---|---|---|---|---|
| | | 0 h lapsed | 6 h after | 12 h after |
| Streptococcus mutans type G | Example 5 | 6.8×10⁶ | <1 | <1 |
| | Example 6 | 6.8×10⁶ | <1 | <1 |
| | Reference Example 1 | 6.8×10⁶ | <1 | <1 |
| | Comparative Example 2 | 6.8×10⁶ | 1.7×10⁵ | 2.0×10⁵ |
| Streptococcus sanguis | Example 5 | 4.0×10⁶ | 7.5×10² | <1 |
| | Example 6 | 4.0×10⁶ | 1.6×10³ | <1 |
| | Reference Example 1 | 4.0×10⁶ | 3.8×10³ | <1 |
| | Comparative Example 2 | 4.0×10⁶ | 3.7×10⁶ | 4.5×10⁶ |
| Actinobacillus actinomycetemcomitans Y4 | Example 5 | 3.7×10⁵ | <1 | <1 |
| | Example 6 | 3.7×10⁵ | 2.4×10² | <1 |
| | Reference Example 1 | 3.7×10⁵ | 1.5×10 | <1 |
| | Comparative Example 2 | 3.7×10⁵ | 3.5×10⁵ | 4.6×10⁵ |
| Bacteroides gingivalis | Example 5 | 6.5×10⁵ | 5.4×10 | <1 |
| | Example 6 | 6.5×10⁵ | 8.3×10² | <1 |
| | Reference Example 1 | 6.5×10⁵ | 3.1×10³ | <1 |
| | Comparative Example 2 | 6.5×10⁵ | 6.7×10⁵ | 5.2×10⁵ |

As can be appreciated from the test result of Table 2, the compositions of the tooth pastes of the above Examples exhibit a sufficient antibacterial effect against the bacilli which cause carious tooth and periodontal diseases. Additionally, as a result of further tests conducted in which the content or blended ratio of the antibacterial hydroxylapatite was changed in the above specimen tooth pasta of Examples 5 and 6, it was revealed that a sufficient antibacterial effect was already exhibited if 0.01% by weight of the antibacterial hydroxylapatite were added to the tooth paste.

### [Antibacterial Test (II)]

In this test, the antibacterial effect of the antibacterial hydroxylapatite was measured. The test was conducted on the antibacterial hydroxylapatites used in Examples 5 and 6 as follows: First, a binder was added to and mixed with the antibacterial hydroxylapatite to form a mixture. The mixture was subjected to compression-molding and then degreased at 500 °C to thereby obtain a pellet (formed of the antibacterial hydroxylapatite 100%) having a diameter of 3 cm. Thereafter, escherichia coli (E. coli) was adhered to the, pellet, upon which the change of the number of living bacilli with the lapse of time was observed. The test result is shown in Table 3.

**TABLE 3**

| Bacillus | Lapsed time and number of living bacilli | |
|---|---|---|
| Escherichia coli | 0 h | 48 h |
| | 6.8×10⁴ | 10 or less |

### [Released Metal Content Test]

In this test, the released amount of the carried antibacterial metal was measured for a first sample of the hydroxylapatite carrying 5% by weight of silver (Ag) as the antibacterial metal, and for a second sample of the hydroxylapatite carrying 5% by weight of zinc (Zn) as the antibacterial metal. The test was conducted as follows: First, 1 g of each of the first and second sample hydroxylapatites was added to 100 cm³ of distilled water and stirred for a long period of time. Then, measurement was made on the concentration of the metal released from the hydroxylapatite to the aqueous solution. As a result, in case of the first sample, the concentration of the released metal was 0.01 ppm or less. In case of the second sample, the concentration was 0.2 ppm or less. Accordingly, it was confirmed that the antibacterial metal carried by the hydroxylapatite was not released at all to water. Additionally, it was also confirmed that no change occurred in the concentration of the released metal even upon lapse of time. This revealed that the antibacterial hydroxylapatite was highly stable as a molecule, which seemed to be caused because of the fact that the antibacterial metal was indeed firmly combined with or taken up into the fine pores of the molecule structure of the hydroxylapatite.

The antibacterial hydroxylapatite has functions to separate protein and to remove dental plaque as a source for producing carious tooth, and therefore has a further effect of preventing occurrence of the carious tooth in addition to the above-discussed antibacterial effect. Moreover, the antibacterial hydroxylapatite functions to be filled in fine grooves or depressions on the surface of teeth thereby accomplishing the restoration of the teeth.

As appreciated from the above, the antibacterial hydroxylapatite is stable in molecular structure and therefore is prevented from exhibition of toxicity due to the antibacterial metal, maintaining a good preservability for a long period of time. Thus, the dentifrice (tooth paste) containing the antibacterial hydroxylapatite, according to the present invention is very effective for preventing occurrence of carious tooth and periodontal diseases, suppressing occurrence of hyperesthesia due to the carious tooth and the periodontal diseases and other oral disorders in general.

## Claims

1. A dentifrice comprising:
at least one calcium compound selected from the group consisting of calcium hydrogenphosphate, tricalcium phosphate, calcium diphosphate, calcium carbonate, and apatite, said calcium compound being in the form of a powder; and
at least one antibacterial metal selected from the group consisting of silver, copper and zinc, which is carried by said calcium compound powder by ion exchange and/or adsorption.

2. The dentifrice of claim 1, wherein said apatite is a hydroxylapatite.

3. The dentifrice of claim 2, wherein said hydroxylapatite is in the form of a powder.

4. The dentifrice of claim 1, wherein said antibacterial metal is contained in an amount of not more than 30% by weight, relative to said calcium compound.

5. The dentifrice of claim 4, wherein said antibacterial metal is contained in an amount ranging from 0.0001 to 5% by weight relative to said calcium compound.

## Patentansprüche

1. Zahnpflegemittel umfassend:
mindestens eine Calciumverbindung, gewählt aus der Gruppe, bestehend aus Calciumhydrogenphosphat, Tricalciumphosphat, Calciumdiphosphat, Calciumcarbonat und Apatit, wobei die Calciumverbindung in Form eines Pulvers vorliegt; und
mindestens ein antibakterielles Metall, gewählt aus der Gruppe, bestehend aus Silber, Kupfer und Zink, welches auf die pulverförmige Calciumverbindung durch Ionenaustausch und/oder Adsorption aufgebracht ist.

2. Zahnpflegemittel nach Anspruch 1, worin der Apatit ein Hydroxylapatit ist.

3. Zahnpflegemittel nach Anspruch 2, worin der Hydroxylapatit in Form eines Pulvers vorliegt.

4. Zahnpflegemittel nach Anspruch 1, worin das antibakterielle Metall in einer Menge von nicht mehr als 30 Gew.-%, bezogen auf die Calciumverbindung, enthalten ist.

5. Zahnpflegemittel nach Anspruch 4, worin das antibakterielle Metall in einer Menge im Bereich von 0,0001 bis 5 Gew.-%, bezogen auf die Calciumverbindung, enthalten ist.

## Revendications

1. Dentifrice comprenant :
au moins un composé de calcium choisi dans le groupe consistant en hydrogénophosphate de calcium, phosphate tricalcique diphosohate de calcium, carbonate de calcium et apatite, ledit composé de calcium étant sous la forme d'une poudre ; et
au moins un métal antibactérien sélectionné dans le groupe consistant en argent, cuivre et zinc, qui est porté par ladite poudre du composé de calcium par échange d'ions et/ou adsorption.

2. Dentifrice de la revendication 1, où ladite apatite est une hydroxylapatite.

3. Dentifrice de la revendication 2, où ladite hydroxylapatite est sous la forme d'une poudre.

4. Dentifrice de la revendication 1, où ledit métal antibactérien est contenu en une quantité ne dépassant pas 30% en poids relativement audit composé de calcium.

5. Dentifrice de la revendication 4, où ledit métal antibactérien est contenu en une quantité comprise entre 0,0001 et 5% en poids relativement audit composé de calcium.
